# EUROPEAN PATENT APPLICATION

(11) **EP 0 812 586 A1**
(43) Date of publication of application: **17.12.1997**
(21) Application number: 97109362.0
(22) Date of filing: 10.06.1997
(51) Int. Cl.: A61K 7/42, A61K 7/48

(54) **Self-tanning cosmetic compositions containing dihydroxyacetone**

(30) Priority: 11.06.1996 GB 9612160
(71) Applicant: The Boots Company PLC, Nottingham, Nottinghamshire NG2 3AA (GB)
(72) Inventor: O'Neill, Sharon, Nottingham. NG2 3AA (GB)
(74) Representative: Thacker, Michael Anthony

(57) **Abstract**

There is disclosed an artificial tanning composition, preferably in the form of a gel, comprising a cosmetically effective amount of dihydroxyacetone (DHA) as an artificial tanning agent, an alcoholic phase and a hydroxypropylcellulose thickener. The composition may further comprise a glycol, preferably selected from one or more of ethoxydiglycol, propylene glycol and butylene glycol. The composition may further comprise ingredients miscible with oil. Preferably the composition is substantially transparent to visible light, and may further comprise a colouring agent, which is preferably oil soluble, to tint the composition.

There is also disclosed a method of preparing artificial tanning compositions comprising the steps of:
a) dissolving dihydroxyacetone in a minimum amount of water,
b) adding alcohol to the aqueous solution from step a),
c) dispersing hydroxypropylcellulose in the alcoholic mixture from step b),
d) optionally, heating and then cooling to ambient temperature any remaining ingredients; and
e) optionally, adding the remaining ingredients treated as in step d) to the alcoholic mixture from step c).

## Description

The present invention relates to compositions that produce tanned skin in the absence of UV radiation, in particular to those comprising dihydroxyacetone as the tanning agent.

Known artificial tanning compositions use the artificial tanning agent dihydroxyacetone (also referred to herein as 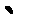 DHA'). The term 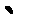 artificial tanning agent' is used herein to denote an agent which acts on the skin to produce a skin tan without exposure to ultra-violet (UV) radiation. This is in contrast with agents that act to accelerate the natural tanning processes that occur when skin is exposed to UV.

DHA reacts with the amino acids and amino groups of keratin proteins present in the outer layers of the skin epidermis, to form brown-coloured compounds. The reaction is believed to be similar to the Mallard reaction (non-enzymatic browning) in which reducing sugars react with amino acids, peptides and proteins to form compounds containing amino and carbonyl functionality, these compounds being polymerised to brown melanoidines.

Artificial tanning agents have the advantage of producing a skin tan without the need to expose the skin to UV radiation. The increasing tendency of people to sunbathe by exposing skin for long periods to UV radiation from sunlight or sunlamps in order to produce a skin tan, has led to an increase in the incidence of skin cancer world-wide, particularly amongst fair skinned people. Depletion of atmospheric ozone has also increased the intensity of UV radiation reaching the earth particularly at higher latitudes. Thus, the use of artificial tanning agents to produce tanned skin is increasingly advantageous, in all climates, as an alternative or supplement to exposure of the skin to UV radiation.

Current artificial tanning compositions comprising DHA are not completely satisfactory. DHA is a difficult material to formulate and it is difficult to produce stable DHA compositions especially those which have some or all of the following advantageous properties. For an artificial tanning composition it is desirable that the composition has good spreading properties to obtain an even coverage, has a good skin feel and penetrates the skin surface easily to provide a longer lasting tan. It is also desirable that the formulation has good resistance to water. In addition artificial tanning compositions which are clear (i.e. substantially transparent to visible light) and which can be tinted in various colours, are desirable as they provide a distinctive and aesthetically pleasing appearance to the consumer. Therefore it is an objection of the present invention to formulate improved DHA compositions.

Surprisingly the applicant has found that a stable artificial tanning composition with some or all of the aforementioned desirable properties can be formed by use of a particular thickening ingredient in an alcoholic phase.

Therefore broadly in accordance with the present invention there is provided an artificial tanning composition comprising a cosmetically effective amount of dihydroxyacetone as an artificial tanning agent, an alcoholic phase and a hydroxypropylcelluose thickener. Preferably the composition comprises a gel.

The amount of DHA may be formulated to suit light, medium or dark skin types. Preferably the DHA comprises from about 0.1 % to about 10 %, more preferably from about 1 % to about 7 %, most preferably from about 2 % to about 5 % by weight of the composition.

The alcoholic phase may comprise any suitable volatile alcoholic liquid compatible with DHA (such as denatured ethanol, t-butyl alcohol and / or any suitable mixtures thereof) together with the minimum amount of water required to dissolve the DHA. Optionally the alcoholic phase may further comprise an ingredient to prevent accidental ingestion of the composition, such as denatonium benzoate (available commercially under the trade name Bitrex).

Preferably the alcoholic phase comprises from about 0.5 % to about 95 %, preferably from about 30 % to about 95 %, most preferably from about 50 % to about 95 % and especially from about 65 % to about 90 %, by weight of the composition.

Preferably the hydroxypropylcellulose comprises from about 0.01 % to about 10 %, more preferably from about 0.1 % to about 5 %, most preferably from about 0.5 % to about 3 % by weight of the composition.

Compositions of the present invention are preferably in the form of a gel, as gels have a good feel on the skin and tend to penetrate the skin more easily which aids production of a longer lasting tan.

Preferably the composition further comprises a glycol ingredient. The term 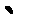 glycol ingredient' is defined herein to mean an ingredient comprising one or more organic compounds each having at least two hydroxy groups. Preferably the glycol ingredient comprises one or more of: ethoxydiglycol, propylene glycol, or butylene glycol; more preferably ethoxydiglycol. Preferably the glycol ingredient comprises from about 0.1 % to about 25 %, more preferably from about 2 % to about 7 %, and most preferably about 5 % by weight of the composition.

It has been found that the addition of a glycol ingredient to artificial tanning compositions is advantageous due to the action of the glycol ingredient as an agent to aid penetration of the skin by the artificial tanning agent. Such tanning compositions produce a tan of an increased colour intensity, with a more natural colour which is less yellow and more red and which lasts longer before fading. The more natural skin tone produced may be due to the reaction of the DHA with proteins associated with the deeper layers of the stratum corneum and / or epidermis. Compositions without a glycol ingredient may only effect proteins in the superficial skin layers.

Compositions of the present invention may further comprise one or more ingredients miscible with oil, preferably as an oil phase. Compositions of the invention may comprise oil in an alcoholic solution and / or an oil-in-alcohol emulsion.

The optional oil miscible ingredients preferably comprise any ingredients which would not render the composition opaque. Suitable oil-miscible ingredients may comprise one or more of:
a) hydrocarbon oils such as paraffin or mineral oils;
b) waxes such as beeswax or paraffin wax;
c) natural oils such as sunflower oil, apricot kernel oil, shea butter of jojoba oil;
d) silicone oils such as dimethicone, cyclomethicone or cetyldimethicone;
e) fatty acid esters such as isopropyl palmitate or isopropyl myristate;
f) alcohols such as fatty alcohols (for example cetyl alcohol or stearyl alcohol) or isopropyl alcohol; and
g) any suitable mixtures thereof.

The oil-miscible ingredients may comprise from about 0.5 % to about 95 %, preferably from about 1 % to about 60 %, more preferably from about 5 % to about 30 %, and most preferably from about 10 % to about 20 % by weight of the composition.

Optionally emulsifiers, surfactants and / or detergents may be used in the compositions of the present invention, for example to provide a cleaning action to the composition or to stabilise the composition if it comprises an emulsion. Preferably such ingredients will not render the composition opaque, more preferably are liquid, and most preferably are clear liquids. Suitable cosmetically acceptable emulsifiers, surfactants and / or detergents known in the art may be comprise one or more of:
a) ethoxylated esters of derivatives of natural oils such as a polyethoxylated ester of hydrogenated castor oil (available commercially from ICI under the trade name Arlacel 989);
b) silicone emulsifiers such as silicone polyols (available commercially from Th. Goldschmidt AG under the trade name ABIL WS05 and from Dow Corning under the trade designation Silicone Fluid 3225C);
c) sorbitan esters (available commercially from Croda under the trade name Crill);
d) ethoxylated sorbitan esters (available commercially from ICI under the trade name Tween); and
e) any suitable mixtures thereof.

The amount of emulsifier, surfactant and / or detergent optionally present in the compositions of present invention is preferably in the range from about 0.1 % to about 20 % by weight of the composition.

Compositions of the present invention may also comprise colouring agents, such as tints, colours and / or dyes, to colour the composition. Preferably the colouring agents comprise oil soluble colours. Any suitable colouring agents may be used which are compatible with the current legalisation in place in the relevant territory in which the composition is to be manufactured, sold and / or used.

Artificial tanning compositions of the present invention are preferably substantially transparent to visible light and preferably also substantially free from cloudiness or turbidity.

It also has been discovered that in the presence of iron salts, DHA produces an artificial tan less yellow in colour. This is described in the applicant's European patent application 0688203. Therefore preferably the DHA compositions of the present invention may also comprise one or more iron salts. The presence of iron also causes an immediate pale colour to be produced when the DHA is initially applied to the skin. This aids topical application of the composition in an even layer which avoids a streaky appearance in the resultant artificial tan.

Compositions of the present invention may also comprise sunscreen agents so the artificial tanning composition also acts as a sunscreen. As used herein the term 'sunscreen agent' denotes an active ingredient which is used to protect the skin against incident UV radiation whether by absorbance, reflection or otherwise. The term 'sunscreen' is used herein to encompass compositions such as moisturisers, day creams, tanning lotions and sun-blockers which are intended for topical application to provide protection against the sun's rays or other sources of ultraviolet (UV) radiation.

Preferably sunscreens of the present invention comprise sunscreen agents which would not render the composition opaque. Suitable sunscreen agents comprise organic sunscreen agents such as one or more of:
a) p-aminobenzoic acids, esters and derivatives thereof, for example:
   ethylhexyl p-dimethylaminobenzoate; and
   the octyl ester of p-aminobenzoic acid;
b) methoxycinnamate esters, for example:
   2-ethylhexyl p-methoxycinnamate,
   2-ethoxyethyl p-methoxycinnamate, and
   di-(p-methoxycinnamoyl)-'-(2-ethylhexanoyl) glycerin;
c) benzophenones, for example:
   oxybenzone;
d) dibenzoylmethanes, for example:
   butylmethoxydibenzoyl methane;
e) salicylate esters;
f) sulphonic acid and its derivatives, for example:
   sodium hydroxymethoxybenzophenone sulphonate;
   2-phenylbenzimdazole-5-sulphonic acid; and
   terephthalylidene dicamphor sulphonic acid (available commercially from L'Oreal under the trade name Meroxyl);
g) camphors, for example:
   3-(4-methylbenzylidene)camphor;
h) acrylates, for example:
   2-ethylhexyl-2-cyano-3,3-diphenyl acrylate; and
f) any suitable mixtures thereof.

The sunscreen agent may be present in a sunscreen of the present invention in an amount from about 0.1 % to about 10 % by weight of the composition.

Compositions of the present invention may additionally include one or more other components which will be well known to those skilled in the art, for example:
a) stabilisers to aid the stability of the composition, such stabilising salts, for example sodium chloride, sodium citrate or magnesium sulphate, preferably in an amount from about 0.1 % to about 5 % by weight of the composition;
b) humectants such as glycerin or 1,3 butylene glycol, preferably in an amount from about 0.1 % to about 30 % by weight of the composition;
c) sequestrants such as tetra sodium ethylene diamine tetra acetate dihydrate (available commercially from Rhône Poulenc under the tradename Sequestene NA4), preferably in an amount from a trace amount to about 1 % by weight of the composition, sequestrants not being included in any composition that comprises an iron salt;
d) anti-oxidants such as tocopherol, DL-α-tocopherol acetate or butylated hydroxytoulene, preferably in an amount from a trace amount to about 2 %, more preferably from a trace amount to about 1 %, by weight of the composition;
e) emollients such as mineral oil, polymethylsiloxane, sweet almond oil, petroleum jelly, isopropyl myristate or triglycerides of fatty acids (for example lauric triglyceride, capric/caprylic triglyceride, or mixed triglycerides [available commercially from Huls UK Ltd under the trade name Miglyol 810]), preferably in an amount from about 0.1 % to about 30 % by weight of the composition;
f) moisturisers such as D-panthenol, preferably in an amount from a trace amount to about 1 % by weight of the composition;
g) agents for adjusting pH (and / or its equivalent in non-aqueous systems) such as sodium citrate or potassium hydroxide, preferably in an amount from a trace amount to about 1 % by weight of the composition;
h) film formers to assist spreading on the surface of the skin, such as alkylated polyvinylpyrrolidones, preferably in an amount from a trace amount to about 1 % by weight of the composition;
i) perfumes; and
j) any suitable mixtures thereof.

In a further aspect of the present invention there is provided a method of preparing the artificial tanning compositions described herein, comprising the steps of:
a) dissolving dihydroxyacetone in a minimum amount of water,
b) adding alcohol to the aqueous solution from step a),
c) dispersing hydroxypropylcellulose in the alcoholic mixture from step b),
d) optionally heating and then cooling to ambient temperature any remaining ingredients, and
e) optionally adding the remaining ingredients treated as in step d) to the alcoholic mixture from step c).

The invention will now be illustrated by the following non-limiting examples. Various trade names and the like known to those skilled in the art are used to describe ingredients used in the Examples listed below. For convenience the chemical composition of these ingredients will now be described.
*Arlamol E* is a PPG-15 stearyl ether.
*Carbitol* is an ethoxydiglycol.
*Denatured ethanol B GDE* comprises ethanol, t-butyl alcohol and denatonium benzoate (*Bitrex*).
*Dermacryl* 79 is an acrylates / t-octylacrylamide copolymer.
*Klucel MF* is a hydroxypropylcellulose thickener.
*Parsol MCX* is an octyl methoxycinnamate sunscreen.
*Parsol 1789* is a butyl methoxydibenzoylmethane sunscreen.
*Transcutol* is an ethoxydiglycol penetration aid.
*Uvinul M 40* is a benzophenone-3 sunscreen.

The terms '% w/w' or 'by weight' as used herein and in the Examples are synonymous and denote the amount of an ingredient present in a composition (expressed as a percentage fraction) calculated from the amount of mass (or weight) of the ingredient compared to the total mass (or weight) of the composition.

### Example 1 (artificial tanning clear tinted gel)

An artificial tanning gel comprising DHA was prepared from the following ingredients using the method described below:

| *Ingredient* | *% w/w* |
|---|---|
| Purified water BP | 2.5 |
| Dihydroxyacetone (tanning agent) | 1 |
| Denatured ethanol B GDE (alcoholic phase) | 68.432 |
| Klucel MF (thickener) | 1.5 |
| Parsol MCX (organic sunscreen) | 6.5 |
| Parsol 1789 (organic sunscreen) | 3 |
| Dioctyl maleate (solvent for sunscreens) | 15 |
| Transcutol (skin penetration aid for DHA) | 2 |
| Red No 17 D & C (oil soluble colour) | 0.03 |
| Yellow No 11 D & C (oil soluble colour) | 0.03 |
| Green No 6 D & C (oil soluble colour) | 0.008 |
| Perfume | qs |
| Preservative | qs |

The DHA was dissolved in the water and the ethanol was added to this aqueous solution. The Klucel MF was added to the ethanol mixture and dispersed for 20 minutes. The remaining ingredients were mixed together and heated to 70 - 75 °C and then force cooled to 30 °C. These ingredients were added to the ethanol mixture which was then stirred for 10 minutes.

The resultant artificial tanning composition was a stable, transparent, water-resistant, tinted gel with a Sun Protection Factor (SPF) of 8 and a 3 star rating according to the Boots star rating system.

### Example 2 (artificial tanning clear tinted gel)

An artificial tanning gel comprising DHA was prepared from the following ingredients using the method described below:

| *Ingredient* | *% w/w* |
|---|---|
| Purified water BP | 2.5 |
| Dihydroxyacetone (tanning agent) | 1 |
| Denatured ethanol B GDE | 62.604 |
| Triethanolamine 80 % pure solution | 0.9 |
| Dermacryl 79 | 2 |
| Klucel MF (thickener) | 1.5 |
| Parsol MCX (organic sunscreen) | 6.5 |
| Parsol 1789 (organic sunscreen) | 3 |
| Uvinul M 40 (organic sunscreen) | 2 |
| C₁₂-C₁₅ alcohols benzoate | 12.6 |
| Arlamol E | 3 |
| Dl-α-tocopheryl acetate (anti-oxidant) | 0.02 |
| Red No 17 D & C (oil soluble colour) | 0.03 |
| Yellow No 11 D & C (oil soluble colour) | 0.03 |
| Green No 6 D & C (oil soluble colour) | 0.008 |
| Carbitol | 2 |
| Perfume | 0.3 |

The DHA was dissolved in the water and the ethanol and triethanolamine were added to this aqueous solution. The Dermacryl 79 was added to the ethanol mixture and dispersed for 10 minutes then the Klucel MF was added to the ethanol mixture and dispersed for 15 minutes. The remaining ingredients (except the Carbitol and the perfume) were mixed together and heated to 70 - 75 °C and then force cooled to 30 °C. This mixture were added to the ethanol mixture and the resultant mixture was then stirred for 10 minutes. The Carbitol was then stirred into this mixture and if necessary the composition was made to weight with more ethanol.

The resultant artificial tanning composition was a stable, transparent, water-resistant, tinted gel with a Sun Protection Factor (SPF) of 8 and a 3 star rating according to the Boots star rating system.

## Claims

1. An artificial fanning composition comprising a cosmetically effective amount of dihydroxyacetone as an artificial tanning agent, an alcoholic phase and a hydroxypropylcellulose thickener.

2. An artificial fanning composition as claimed in claim 1, which comprises a gel.

3. An artificial fanning composition as claimed in either claim 1 or 2, which comprises a glycol ingredient.

4. An artificial fanning composition as claimed in claim 3, in which the glycol ingredient comprises one or more of: ethoxydiglycol, propylene glycol and butylene glycol.

5. An artificial fanning composition as claimed in any preceding claim, which comprises one or more ingredients miscible with oil.

6. An artificial tanning composition as claimed in any preceding claim, in which the composition further comprises a colouring agent.

7. An artificial tanning composition as claimed in claim 6, in which the colouring agent is substantially soluble in oil.

8. An artificial tanning composition as claimed in any preceding claim, in which the composition is substantially transparent to visible light.

9. A method of preparing an artificial tanning composition comprising the steps of:
a) dissolving dihydroxyacetone in a minimum amount of water,
b) adding alcohol to the aqueous solution from step a),
c) dispersing hydroxypropylcellulose in the alcoholic mixture from step b),

10. A method of preparing an artificial tanning composition as claimed in claim 9, comprising the additional steps of:
d) heating and then cooling to ambient temperature any remaining ingredients; and
e) adding the remaining ingredients treated as in step d) to the alcoholic mixture from step c).
